Europäisches Patentamt

European Patent Office

Office européen des brevets

(19)

(11) Publication number: **0 229 442 B1**

## EUROPEAN PATENT SPECIFICATION

(12)

(45) Date of publication of patent specification: **27.01.93**

(51) Int. Cl.5: **C12Q 1/68**

(21) Application number: **86304429.3**

(22) Date of filing: **10.06.86**

(54) Accelerated nucleic acid reassociation method.

(30) Priority: **07.01.86 US 816711**

(43) Date of publication of application:
**22.07.87 Bulletin 87/30**

(45) Publication of the grant of the patent:
**27.01.93 Bulletin 93/04**

(84) Designated Contracting States:
**AT BE CH DE FR GB IT LI LU NL SE**

(56) References cited:
**EP-A- 0 127 327**
**EP-A- 0 133 288**
**US-A- 4 302 204**
**US-A- 4 533 628**

**BIOCHEMISTRY, vol. 16, no. 24, 1977, pages 5329-5341, US; D.E. KOHNE et al.: "Room temperature method for increasing the rate of DNA reassociation by many thousandfold: The phenol emulsion reassociation technique"**

(73) Proprietor: **GEN-PROBE INCORPORATED**
**9880 Campus Point Drive**
**San Diego California 92121-1514(US)**

(72) Inventor: **Kohne, David**
**364 Nautilus**
**La Jolla California 92037(US)**
Inventor: **Kacian, Daniel**
**3911 Tambor Road**
**San Diego California 92124(US)**

(74) Representative: **Goldin, Douglas Michael et al**
**J.A. KEMP & CO. 14, South Square Gray's Inn**
**London WC1R 5EU(GB)**

**Description**

BACKGROUND OF THE INVENTION

Field of The Invention

The present invention is directed to a method for the renaturation, reassociation or hybridization of single stranded nucleic acid molecules into double stranded nucleic acid molecules wherein the rate of reaction is greatly increased over the rate of reaction under standard reference conditions of 0.12M phosphate buffer at 60°C. More particularly, the present invention is directed to a method for the renaturation, reassociation or hybridization of nucleic acids, including DNA to DNA, RNA to DNA, and RNA to RNA reactions wherein the rate of the reaction is greatly increased by factors of 50 to 100 times, and even up to several thousand times that of the reaction rates observed under standard reference conditions. These greatly accelerated reaction rates are achieved through the utilization of reaction solutions containing nucleic acid precipitating agents.

Description of The Prior Art

Numerous methods for the nucleation of single stranded nucleic acid molecules into double stranded molecules are known in the art and have proven to be useful tools for the analysis of genetic material from a wide variety of organisms. Generally speaking, these nucleation reactions, renaturation, reassociation and hybridization, are based on the tendency of single stranded nucleic acid molecules having blocks or segments of complementary base sequences to form base paired structures between these complementary sequences and to rewind forming double helices. The greater the extent of sequence complementarity between the single stranded nucleic acid molecules, the greater the tendency for a given pair of molecules to nucleate and form a double stranded or duplex molecule.

Renaturation, reassociation and hybridization are essentially synonymous reactions between single stranded nucleic acid molecules. As such, they will be discussed interchangeably throughout the body of this paper. However, the following distinction may prove helpful in understanding the technology involved. Renaturation generally refers to the formation of a double stranded nucleic acid molecule from two single stranded molecules which were originally base paired to one another and separated through a denaturation process. Reassociation refers to the process of forming double stranded nucleic acid molecules between two single stranded molecules which usually come from different original molecules. Hybridization refers to the formation of double stranded nucleic acid molecules from single stranded nucleic acid molecules of individual origin with respect to one another. It should be appreciated that these are not clear cut distinctions and that there is considerable overlap between them. For example, DNA:DNA reactions are commonly called both reassociation and hybridization reactions. On the other hand, the formation of an RNA:RNA double stranded molecule is generally referred to as hybridization.

The kinetics of these reactions are well understood in the art also following second-order kinetics. Thus, as the concentration of the single stranded nucleic acid molecules is increased, the rate of the reaction is also increased. Conversely, decreasing the concentration of the single stranded nucleic acid reactant will decrease the rate of reaction and thus increase the time necessary for the formation of the double stranded nucleic acid molecules to take place.

The effect of temperature on the reaction rate is also well known in the art. As the temperature of the reaction decreases below the $T_m$ (the temperature at which 50% of the double stranded molecules is denatured, also known as the "melting temperature") a maximum rate for the reaction is achieved at temperatures of approximately 15°C to 30°C below the $T_m$. Further decreases in temperature are known to decrease the rate below this maximum rate.

Lastly, with respect to the kinetics of these reactions, it is known that the reaction rates are very dependent on the ionic strength below 0.4M for electrolytes such as NaCl and are almost independent of the salt concentration above this ionic strength.

More information on the kinetics and reaction rates of these nucleic acid association reactions can be found in the following publications:

Wetmur, R., and Davidson, N. (1968), J. Molec. Biol. 31, 349;

Wetmur, R. (1975), Biopolymers 14, 2517;

Britten, R., J., Graham, D., and Neufeld, B. (1974), Methods Enzymol, 29, 363;

Kohne, D. E., Levinson, S.A., and Byers, M. J. (1977) Biochemistry 16, 5329; and

Orosz, J. M., and Wetmur, J. G., (1977) Biopolymer 16, 1183.

It has long been recognized in the art that a major limitation on the utility of these known nucleic acid association techniques is the basic rate of the reaction. Reaction times on the order of several hours to tens of hours and even days are commonplace. Increasing the reaction rate by increasing the quantities of single stranded nucleic acid molecules utilized in the reactions (due to the second-order kinetics) is not a desirable solution to this problem for three reasons. First, in many cases the target single stranded nucleic acid in the reaction is extracted from physiological samples which inherently limits the amount of such nucleic acid available to that contained in the cells of the physiological sample. Secondly, there are significant expenses associated with the use of nucleic acid reactants which limits the practical utility of increasing the quality of reactants. Thirdly, increasing the quantities of single stranded nucleic acid molecules decreases the sensitivity of the reaction by increasing the background noise. Nonetheless, a number of techniques have been developed to increase the basic rate of these reactions by factors of 5 to 50 or more. Techniques of limited applicability have also been developed which increase the basic reaction rate by factors on the order of 1000 or more. However, as will be discussed in detail below, none of these prior art techniques has been successful at producing greatly accelerated reaction rates of 50 to 100 times or more than the basic reference standard reaction in a single phase system applicable to DNA:DNA, DNA:RNA, and RNA:RNA reactions.

When dealing with reaction rates, the accepted standard reference condition for the comparison of these rates is an aqueous solution of 0.12M phosphate buffer (PB) at 60°C. A similar standard reference condition that is often used giving comparable reaction rates is an aqueous solution of 0.18M NaCl at 60°C.

By far the most common technique of accelerating the reaction rate above that of the standard reference condition has been to increase the salt concentration of the reaction solution above that of the standard reference condition. As detailed in following table, while significant reaction rate increases are observed by increasing the salt concentration, the prior art techniques indicate that the rate of increase levels off or even falls for salt concentrations above 2M.

## TABLE 1

### PRIOR ART KNOWLEDGE OF EFFECT OF
### SALT CONCENTRATION ON DNA:DNA HYBRIDIZATION RATES

| Salt | Rate increase relative to 0.18M NaCl reference condition |
|---|---|
| A.  Sodium Chloride | |
| 0.18M | 1 |
| 0.72M | 5.8 |
| 1M | 7 |
| 1.2M | 7.7 Britten, et al. |
| 1.85M | 8.6 |
| 3.2M | 12.3 |
| 4.75M | 21 |
| B.  Cesium Chloride | |
| 1M | 7.6 |
| 4M | 12.7 |
| 7.5M | 15.6 |

4

C.  Sodium Phosphate

0.12(0.18M Na)          1

0.48M (0.72M Na)        5.6 Britten, et al. and

1M (1.5M Na)            8.4 Wetmur and Davidson

1.23M (1.85M Na)       10.1

2.1 (3.2M Na)          12.1


D.  Sodium Perchlorate

1M                     11

2.2M                    6.8 Wetmur and Davidson

4.0M                    3.4

5.2M                    1.5

6.4M                    0.7


E.  Lithium Chloride

0.4M                    3.9 Orosz and Wetmur

1M                     11.6


F.  Potassium Chloride

0.7M                    5.3

1M                      5.8

2M                      5.4 Orosz and Wetmur

3M                     10.0

4M                     11


G.  Sodium Bromide

3M                      9 Orosz and Wetmur


H.  Sodium Sulfate

3M                      9 Orosz and Wetmur

I.   Ammonium Chloride

4M                                  30

While the data in Table 1 relates to the rate increases found with respect to DNA:DNA reactions, it will be appreciated that the reaction rates of RNA with DNA are reported as being less affected by changes in salt concentration. Other researchers have demonstrated that for salt concentrations above the standard reference conditions, the relative rate of RNA:DNA reaction is affected to about one-half the extent of those rates found for DNA:DNA reactions when the RNA used has comparatively little secondary structure. For RNA reactants with more secondary structure, the effect of elevated salt concentration has been found to be even less. In fact, no change in rate is observed for hybridization of excess RNA with DNA over comparative ranges of salt concentrations (see, e.g., Van Ness, J. and Hahn, W. E. (1982) Nucl. Acids. Res. 10, 8061). While little data is available for RNA:DNA hybridization where the DNA is the excess reactant, it is commonly assumed in the art that the effect of elevated salt concentration on such a reaction system is comparable to that of the excess RNA system.

An alternative approach to the acceleration of the rate of these nucleic acid association reactions is the previously developed two-phase phenol aqueous emulsion technique for the reassociation of DNA to DNA (Kohne, D. E., Levinson, S. A., and Byers, M. J. (1977) Biochemistry 16, 5329). In this two-phase system, the agitation of an emulsion formed between phenol and an aqueous salt solution has produced greatly accelerated reaction rates over 100 times faster than comparative standard condition rates. However, the two-phase phenol emulsion technique has not produced similarly greatly accelerated reaction rates for RNA:RNA and RNA:DNA systems. The greatest reaction rate increase observed for RNA:RNA and RNA:DNA reactions is only 50 to 100 times that of the standard reference condition rate. This technique is further limited in that reaction will not occur unless an emulsion is present and agitated and the reaction temperature is below 75°C.

A number of other techniques for producing reaction rate increases on the order of 10 fold above the standard reference condition rate have utilized the volume exclusion principle to promote the acceleration of the reaction rate. These techniques utilize the synthetic polymers polyethylene glycol, dextran, or dextran sulfate to reduce the volume of reaction solution available to the nucleic acid reactants and thereby increase their effective concentration. However, while reaction rate increases of 10 to 15 fold over the standard reference condition rate for DNA:DNA reactions have been reported, rate increases of only about 3 fold are reported for RNA:DNA reactions. Details of these techniques can be found in the following publications:

Renz, M., and Kurz, C. (1984) Nucl. Acids Res. 12, 3435; and

Wahl, G. M., Stern, M., and Stark, G. R. (1979) Proc. Natl. Acad. Sci. USA 75, 3683.

Accordingly, it is a principal object of the present invention to provide a method for the renaturation, reassociation, or hybridization of nucleic acids that produces a greatly accelerated reaction rate on the order of 100 or more times that of the standard reference condition rate and that is applicable to DNA:DNA, RNA:DNA, or RNA:RNA reaction systems. Additionally, it is a further object of the present invention to provide a method that promotes greatly accelerated reaction rates without requiring the utilization of a two-phase system or the formation of an emulsion. It is a further object of the present invention to provide a method wherein greatly accelerated reaction rates are obtainable without the need to increase the concentrations of single stranded nucleic acid reactants. Lastly, it is an additional object of the present invention to provide a method for greatly accelerating the rate of these nucleic acid association reactions that is widely applicable to a variety of reaction mixture volumes and hybridization temperatures.

SUMMARY OF THE INVENTION

Generally stated, the present invention accomplishes the above described objectives by providing a method for the formation of double stranded nucleic acid molecules from separate single stranded nucleic acid molecules wherein a single phase reaction solution incorporating a known concentration of at least one nucleic acid precipitating agent detergent is utilized to greatly increase the reaction rate over the standard reference condition reaction rate. The improved method of the present invention is widely applicable to a broad range of reaction solution volumes and nucleic acid concentrations and promotes reaction rates on the order of 100 to 1000 fold greater than the standard reference condition reaction rate for DNA:DNA, RNA:DNA and RNA:RNA reactions.

More particularly, the method of the present invention comprises the steps of preparing an aqueous reaction solution containing complementary single stranded nucleic acids, one of which preferably incorporates a detectable marker, and a known concentration of at least one nucleic acid precipitating agent. The aqueous reaction solution so prepared is incubated at a temperature at which hybridization can occur and then assayed for the presence of double stranded nucleic acid molecules.

Additionally, alternative methods of the present invention are disclosed wherein the aqueous reaction solution also contains a known concentration of a nucleic acid denaturing agent and also where the nucleic acid precipitating agent is contained in a second solution which is added to the aqueous reaction solution before the incubation step.

The nucleic acid precipitating agents which are utilized to practice the various alternative methods of the present invention are selected from the group consisting of detergent, e.g. sodium dodecyl sulfate, sodium diisobutyl sulfosuccinate and sodium tetradecyl sulfate. The concentration of the nucleic acid precipitating agent preferably ranges from approximately 5 volume % to 95 volume % and the preferred concentration of the nucleic acid denaturing agents ranges from approximately 5 volume % to 95 volume %.

Incubation temperatures preferably range from just below the $T_m$ of the double stranded nucleic acid association product to temperatures approaching room temperature of approximately 22°C. It will be appreciated that the addition of nucleic acid denaturing agents to the aqueous reaction solution will lower the temperature at which hybridization occurs. The hybridization temperatures for most reactions utilizing the methods of the present invention will range from approximately room temperature to 90°C.

After incubation, the reaction solution is assayed through a variety of known assay techniques to detect the presence of the double-stranded nucleic acid product. A preferred assay procedure utilizes hydroxyapatite (HA) for this purpose.

Further objects, features and advantages of the method of the present invention will become apparent to those skilled in the art from a consideration of the following detailed description. The following abbreviations are offered as an aid to understanding the specification.

## ABBREVIATIONS

| | |
|---|---|
| PB | Sodium phosphate solution. (A mixture of equimolar amounts of $Na_2HPO_4$ and $Na\,H_2PO_4$.) |
| PK | Proteinase K |
| EGTA | Ethylene glycol bis- $\beta$ -aminoethyl ether) N,N,N',N'-tetraacetic acid |
| HA | Hydroxyapatite |
| STDS | Sodium tetradecyl sulfate |
| SDIBSS | Sodium diisobutyl sulfosuccinate |
| Tris | Tris(hydroxymethyl)aminomethane hydrochloride |
| G HCl | Guanidine hydrochloride |
| DNA | Deoxyribonucleic acid |
| RNA | Ribonucleic acid |
| rRNA | Ribosomal ribonucleic acid |
| cDNA | Complementary DNA |
| DTT | Dithiothreotol |
| SDS | Sodium dodecyl sulfate |

## DETAILED DESCRIPTION OF THE INVENTION

In a broad aspect, the method of the present invention is based upon the surprising discovery that relatively high concentrations of nucleic acid precipitating detergents will greatly accelerate the rate at which single stranded nucleic acid molecules with regions of complementary base sequences will combine to form base paired double stranded nucleic acid molecules. Reaction rates are increased as much as 800 times over the standard references condition rate for DNA:DNA reactions and as much as 3000 times over the standard reference condition rate for RNA:DNA reactions and as much as 1000 times over the standard reference condition rate for RNA:RNA reactions. What is more, these greatly increased reaction rates occur in a one-phase system and no emulsion or shaking is required.

Such significant increases in the rate of these reactions comes in complete contrast to the teaching of the prior art. For example, an often used accelerated reaction condition is approximately 1M NaCl (or an equivalent to it) which produces a reaction rate approximately 8 to 25 times faster than the reference standard condition. Such a rate increase is not the "greatly accelerated" rate disclosed by the present invention. As shown by the prior art table discussed above, increasing the concentration of NaCl to 4.75M

EP 0 229 442 B1

results in little more of a rate increase. Similarly, concentrations of CsCl (also commonly used to promote increased reaction rates) up to 7.5M produce analogous increases of approximately 15 times that of the standard reference condition rate. Similar rate increases over the strandard reference condition rate were also found for 1M $(NH_4)_2SO_4$ and 1M LiCl, each salt producing rate increases of approximately 13 fold to 18 fold, which is roughly comparable to the rate increase observed for 1M NaCl or CsCl. However, in contrast to these known results, it was surprisingly discovered that increasing the concentration of $(NH_4)_2SO_4$ to 2M greatly increased the reaction rate by an additional factor of 33 to approximately 600 times that of the standard reference condition rate. Additional research disclosed a comparable rate increase when the concentration of LiCl was increased to 4M wherein a rate increase of an additional 30 fold was observed, producing an accelerated reaction rate 467 times greater than that of the standard reference condition. Comparable patterns of great acceleration were also discovered for ammonium formate, cesium sulfate, sodium sulfate, lithium sulfate, sodium phosphate and several detergents and organic compounds.

These same factors (aggregation or precipitation) which prevented the earlier researchers from discovering the method of the present invention are also proposed as being responsible for the greatly accelerated reaction rates of the methods of the present invention. It is hypothesized that nucleic acid precipitating agents cause the single stranded nucleic acid molecules to aggregate and thereby stimulate the reaction rate. As discussed above, the rate at which a given pair of complementary single stranded nucleic acids will form double stranded nucleic acid molecules is directly related to their concentration in the reaction solution. The higher the nucleic acid concentration, the faster the rate of reaction. In the presence of a nucleic acid precipitating agent, the single stranded nucleic acid molecules aggregate or associate together in solution. This aggregation or semi-precipitation results in high concentrations of nucleic acid in localized regions of the reaction solution. If the aggregation occurs at a temperature where hybridization or reassociation can occur, the rate of the reaction is greatly increased.

In order for the complementary single stranded nucleic acid molecules to reassociate or hybridize together in a reaction solution containing a nucleic acid precipitating agent, the temperature must be high enough for the reaction to occur. Renaturation, reassociation or hybridization usually occurs at optimal rates at roughly 10°C to 30°C below the $T_m$ of the double stranded nucleic acid molecule involved. In the reaction solution of the present invention, the $T_m$ of most double stranded nucleic acid molecules will range from 85°C to 100°C. When the reaction solution of the present invention contains one or more nucleic acid denaturing agents, the $T_m$ will be greatly lowered and temperatures as low as room temperature can be utilized to achieve optimum reaction rates. Accordingly, reaction temperatures of approximately 20°C to 90°C should produce optimum rates of reaction.

As discussed above, organic compounds which are miscible with the reaction solution and which have precipitating or salting out properties are effective in promoting greatly accelerated reaction rates. Examples of such compounds include detergents, such as sodium dodecyl sulfate, sodium diisobutyl sulfosuccinate and sodium tetradecyl sulfate.

To determine which compounds possess the requisite nucleic acid precipitating properties to practice the method of the present invention, it is necessary to first screen the compounds to determine if the compounds will precipitate single stranded nucleic acid molecules and then to determine whether the precipitate so formed will disappear when the reaction solution is heated to a temperature where reaction can occur. The nucleic acid precipitating agents are then analyzed to determine the preferred concentrations and incubation temperatures for producing optimal reaction rate increases. This screening procedure also makes it possible to determine the effective concentration of the nucleic acid precipitating agent necessary to promote greatly increased reaction rates.

For example, Sarkosyl (N-lauroylsarcosine sodium salt) was screened for its ability to increase nucleic acid reassociation rates in the following manner. First, a series of solutions containing known amounts of purified liver RNA (final concentration at 4 mg/ml) and varying amounts of Sarkosyl (ranging from approximately 9 volume % to 24 volume %) were prepared. The solutions were thoroughly mixed and checked for the presence of a precipitate using either direct visual observation or a spectrophotometer using a wavelength at which neither the Sarkosyl or the nucleic acid absorbs. If a precipitate was observed in a solution, the solution was heated to approximately 40°C to 90°C to determine whether the degree of precipitation would change. It was found that at 10% to 14% Sarkosyl, little or no precipitation of the nucleic acid was observed. However, at higher concentrations, Sarkosyl was found to precipitate nucleic acid. A number of further experiments were then conducted to determine the preferred concentration and incubation temperatures for producing optimal reaction rate increases.

The following tables are an illustrative listing of the reaction rate increases that can be expected with a variety of concentrations of preferred inorganic salt nucleic acid precipitating agents.

EP 0 229 442 B1

## TABLE 2

### THE EFFECT OF HIGH CONCENTRATIONS OF
### CERTAIN SALTS ON DNA:DNA HYBRIDIZATION RATE

| Salt | Rate increase relative to 0.18M Na reference condition |
|---|---|
| A. Ammonium Sulfate | |
| 1M | 17.5 |
| 2M | 600 |
| 2.1M | 600 |
| 2.5M | 467 |
| 3.1M | 70 |
| B. Lithium Chloride | |
| 1M | 13 |
| 3.5M | 66 |
| 4M | 467 |
| 5M | 280 |
| 6M | 19 |
| C. Other Salts | |
| 2M Cesium Sulfate | 280 |
| 1.9M Sodium Sulfate | 600 |
| 2M Lithium Sulfate | 420 |
| 6M Ammonium Formate | 210 |
| 2.4M Sodium Phosphate | 800 |

9

## TABLE 3

### EFFECT OF HIGH CONCENTRATIONS OF
### SALTS ON EXCESS RNA:DNA HYBRIDIZATION RATES

| Salt | Rate increase relative to reference 0.18M Na condition |
|---|---|
| 0.18M Sodium Chloride | 1 |
| 0.72M Sodium Chloride | 3.6 |
| 2M Ammonium Sulfate | 1500 |
| 2.4M Sodium Phosphate | 3000 |
| 4M Lithium Chloride | 600 |
| 2M Sodium Sulfate | 3460 |
| Ammonium Sulfate | |
| 1M | 90 |
| 2M | 1500 |
| 3M | 600 |

With this understanding of the nucleic acid precipitating agents, the method of the present invention is as follows. The first step of the preferred method is the preparation of an aqueous reaction solution containing a quantity of a first single stranded nucleic acid molecule and a quantity of a second single stranded nucleic acid molecule, preferably incorporating a detectable marker and at least one segment of base sequences which are complementary to a corresponding segment of base sequences of the first single stranded nucleic acid molecule. Additionally, a known concentration of at least one of the previously discussed nucleic acid precipitating agents is also incorporated into the aqueous reaction solution in a concentration sufficient to greatly accelerate the rate of reaction by a factor of at least 50 to 100 times the rate of the standard reference condition reaction. Outside of practical consideration such as the solubility limit of the single stranded nucleic acid reactants, there is no real limit as to the volume of aqueous reaction solution that may be utilized to practice the method of the present invention or to the quantity of single strand nucleic acid molecule reactants as well. Additionally, while it should be emphasized that the nucleic acid precipitating agent is all that is necessary to obtain the greatly increased reaction rates, additional additives may be incorporated into the aqueous reaction solution such as buffers, EGTA, EDTA, SDS, SK, PK, ETOH, Urea, Guanidine HCL, Glycogen and dilute Amphyl. Additionally, it should be noted that while it is preferred that at least one of the single stranded nucleic acid molecule reactants incorporates a detectable marker, the marker is not essential to promoting the greatly accelerated reaction rates.

The next step of the method of the present invention is to incubate the aqueous reaction solution. As discussed above, temperatures ranging from just below the $T_m$ to approximately room temperature are sufficient for incubating the reaction solution. The actual temperature utilized will vary depending on the concentrations of the reactants and whatever additional additives are incorporated into the reaction solution. However, most reactions will be conducted at incubation temperatures ranging from approximately room temperature to 90°C.

The last step in the method of the present invention is to assay the incubated aqueous reaction solution for the presence of double stranded nucleic acid molecules. A wide variety of assaying techniques are known in the art and are contemplated as being within the scope of the present invention. A preferred assaying technique involves the removal of an aliquot from the incubated reaction solution at a specified time after the start of the reaction. The aliquot is diluted into 1 ml of 0.14M PB, 0.02% sodium dodecyl sulfate (SDS). The diluted solution is then passed over a column of hydroxyapatite (HA) (bed volume equaling 1 ml) which has been preequilibrated to 0.14M PB, 0.02% SDS at 67°C. Single stranded DNA molecules will not bind to the HA, but RNA and double stranded nucleic acid molecules will be adsorbed to the column. Nonhybridized single stranded nucleic acid molecule are then removed from the column by passing 5 ml of column buffer 0.14M PB, 0.02% SDS over the column. The adsorbed nucleic acid is recovered from the column by eluting the column with 0.3M PB at 67°C. The various solution fractions so produced may then be assayed for the detectable marker (such as radioactive hydrogen or iodine).

An alternative preferred method of assaying the incubated aqueous reaction solution for the presence of double strand nucleic acid follows.

(a) Remove an aliquot from the solution to tested and mix with 5ml of 0.14M PB, 0.02% SDS containing one 0.1 gm of HA. Vortex the mixture for 5-10 seconds.

(b) Incubate the mixture at 72°C for 5 minutes.

(c) Centrifuge the mixture in a table top centrifuge for 1 minute to pellet the HA. Discard the supernate fraction.

(d) Add 5ml of 0.14M PB, 0.02% SDS to the tube and vortex to resuspend the HA.

(e) Repeat (c).

(f) Assay the HA for detectable marker.

An alternative approach for practicing the method of the present invention incorporates the additional step of mixing a second solution containing the nucleic acid precipitating agent into the previously prepared aqueous reaction solution prior to incubating the resultant mixture. Thus, the alternative method comprises the steps of preparing the previously discussed aqueous reaction solution, mixing the aqueous reaction solution with a second solution containing a known concentration of at least one nucleic acid precipitating agent which is miscible with the aqueous solution add capable of precipitating single stranded nucleic acid molecules from an aqueous solution, incubating the resulting mixture at the previously discussed temperatures, and assaying the incubated mixture for the presence of double stranded nucleic acid molecules. This alternative method serves to eliminate any problems which may occur with the premature aggregation of the single stranded nucleic acid molecule reactants in the aqueous reaction solution.

An additional modification to both of the alternative methods for practicing the accelerated rate reaction of the present invention involves the addition of a known concentration of at least one nucleic acid denaturing agent such as alcohol to the aqueous reaction solution. Preferably the concentration of denaturing agent added will range from approximately 5% by volume to approximately 95% by volume. For example, alcohol is a denaturant and functions to lower the temperature at which the reaction will occur. Ethanol is soluble in 2M $(NH_4)_2SO_4$ to approximately 20%. At this concentration, the reaction will occur at a temperature of approximately 49°C instead of the usual 60°C to 80°C.

Another denaturing agent which can be added to the aqueous reaction mixture is Urea. The presence of Urea in the reaction mix has little effect on the extent or rate of hybridization in several of the accelerated rate systems checked thus far. Example 17 is an example of one such system. Urea is an excellent solubilizing agent for many non-nucleic acid compounds which may be present in a sample and is useful to minimize any effect these compounds might have on the hybridization reaction. Preferably, the concentration of Urea present in the reaction mixture will be approximately 0.01 to about 4M. The actual amount of Urea to be used must be determined for each different situation.

Guanidine HCl (GHCl) is another denaturing agent which can be added to the aqueous reaction mixture. This agent is useful to solubilize non-nucleic acid substances which may otherwise interfere with the hybridization reaction. Addition of GHCl to an aqueous reaction mixture optimized for both rate and extent of hybridization seen at specific times of incubation. A higher concentration of accelerating agent must be used in order to optimize the extent of hybridization when GHCl is present. Example 26 presents data concerning this. It is likely that the GHCl solubilizes nucleic acids to a certain extent and that more accelerating agent is needed to concentrate the nucleic acids for rapid hybridization. A similar situation occurs with the sodium phosphate system as seen in Example 26.

Regardless of which of the alternative methods is utilized to practice the method of the present invention, the nucleic acid precipitating agents are preferably selected from the group consisting of detergent eg, sodium dodecyl sulfate (SDS), sodium diisobutyl sulfosuccinate (SDIBSS) or sodium tetradecyl sulfate (STDS) It is also contemplated as being within the scope of the present invention to combine various members of this group in a single aqueous reaction solution. Additionally, it is also contemplated as being within the scope of the present invention to utilize a variety of detergent agents in addition to the organic compounds disclosed. Accordingly, those compounds specifically disclosed and claimed in the present invention are those which are currently known to be suitable for practicing the method of the present invention. Analogous compounds are therefore considered to be within the scope of the present invention.

It will be appreciated that the effective concentrations of nucleic acid precipitating agents necessary to practice the method of the present invention will vary with the amount of nucleic acid as well as with the size of the nucleic acid in the reaction solution and the pH of the solution as well as with the presence of other compounds. Accordingly, it is preferred that the concentrations will range from approximately 1M to 10M for the inorganic salt compounds and from approximately 5% by volume to approximately 95% by volume for the organic compounds. Additionally, it is preferred that the pH of the reaction solution will range from approximately 4 to 11.

Lastly, as discussed above, the preferred incubation temperature for the aqueous reaction solution should range from approximately room temperature to approximately 90°C.

The method of the present invention is suitable for bacterial, viral, mammlian and chemically synthesized nucleic acid. The completeness of the reaction will vary depending upon the concentration of the strong nucleic acid precipitating agent as well as on the amount of nucleic acid in the original reaction solution and on the composition of the reaction mixture. At low concentrations of nucleic acid, well over 90% of the single stranded nucleic acid will associate to form double stranded nucleic acid molecules. At higher nucleic acid concentrations, the completeness of the reaction will only be approximately 70% or less even though the rate of the reaction will be greatly increased. It should be noted that at very high concentrations of nucleic acid reactants, the rate of reaction will be accelerated to a lesser degree.

The amount of single stranded nucleic acid molecule reactants present in the aqueous reaction solution can range from an upward extreme approaching the solubility limit of the nucleic acid molecules to a lower extreme on the order of $10^{-9}$ micrograms. Interestingly, the reaction rate increase for high concentrations of DNA:DNA or DNA:RNA reactions is lower than that for low concentrations of DNA:DNA and RNA:RNA reactions. Thus, the method of the present invention is applicable to both high and low concentrations of reactants. Along these lines, preferred reaction solution volumes will be on the order of a milliliter or less to a fraction of a microliter. However, it should be emphasized that other reaction solution volumes are contemplated as being within the scope of the present invention. Additionally, while the presence of small quantities of protein and other cell components will not greatly interfere with the reaction of the method of the present invention, excess heterologous RNA or DNA or other cellular components will slow the reaction rate to various extents as well as affecting the completeness of the hybridization.

Detergents are useful to help minimize the effect of excess cell and other components on the reaction of the method of the present invention. Addition of detergents compatible with individual rate accelerating agents to reaction mixtures is helpful in this regard. Certain detergents greatly accelerate nucleic acid hybridization and are quite useful in this regard. The optimum rate accelerator concentration to be used for different nucleic acids is dependent on the variables discussed above.

It should also be noted that the greatly accelerated reaction rates have been achieved for nucleic acid molecules ranging from approximately 30 bases long to molecules on the order of $10^4$ bases long. However, the method of the present invention is contemplated as being applicable to nucleic acid molecules ranging from short molecules on the order of 10 to 15 bases long to longer molecules in excess of $10^4$ bases long.

The following examples are offered as being illustrative of the method of the present invention and not by way of limitation.

The methods of the invention described in the above examples have great significance in the area of detection and identification of a wide variety of different life forms in various types of samples. The said methods make it possible to detect and identify many life forms with a rapidity and sensitivity heretofore unattainable. The following examples illustrate this.

EXAMPLE 1

Method For Using Sodium Dodecyl Sulfate To Increase DNA:RNA Hybridization Rates

This example shows that a greatly accelerated hybridization rate Occurs at 28.5% ($\frac{W}{V}$) SDS when probe is in excess over purified RNA.

A. Excess Probe Plus Homologous RNA

1. Thoroughly mix:
   1 μl solution containing 01.6x$10^{-4}$ micrograms Legionella Ribosomal RNA (rRNA).
   1 μl 5M Sodium Phosphate Buffer (pH = 6.8)(PB).
   3 μl probe solution containing $10^{-4}$ micrograms of I$^{125}$ - cDNA complementary to about 1/5 of the Legionella rRNA sequence.

95 $\mu$l 30% ($\frac{W}{V}$) sodium dodecyl sulfate in $H_2O$.

2. Incubate the mixture at 72°C and at specific times remove aliquots. Dilute each aliquot and assay for hybridization as described earlier.

This procedure resulted in a rate increase of 100-200 fold over the rate at the reference condition.

EXAMPLE 2

B. Excess Probe Hybridization

This example shows that a greatly accelerated hybridization rate occurs at 31.4% ($\frac{W}{V}$) SDS when probe is in excess over purified RNA.

1. Thoroughly mix:

1 $\mu$l of solution containing $1.6 \times 10^{-4}$ micrograms of Legionella Ribosomal RNA.

6 $\mu$l $H_2O$.

1 $\mu$l 5M Sodium Phosphate Buffer (pH = 6.8)(PB)

2 $\mu$l probe solution containing $10^{-4}$ micrograms of $I^{125}$ - cDNA complementary to about 1/5 of the Legionella Ribosomal RNA sequence.

90 $\mu$l 34.9% ($\frac{W}{V}$) sodium dodecyl sulfate in $H_2O$.

2. Incubate the mixture at 72°C and at specified times remove aliquots. Dilute each aliquot and assay for hybridization as described earlier.

This procedure resulted in a rate increase of 150-200 fold over the rate at the reference condition.

EXAMPLE 3

C. Excess RNA Hybridization:

This example shows that a greatly accelerated hybridization rate occurs at 31.4% ($\frac{W}{V}$) SDS when purified RNA is in excess.

1. Thoroughly mix:

2 $\mu$l solution containing $3.2 \times 10^{-4}$ micrograms Legionella Ribosomal RNA.

1 $\mu$l $H_2O$.

1 $\mu$l 5.0M Sodium Phosphate Buffer (pH = 6.8) (PB).

2 $\mu$l probe solution containing $2.5 \times 10^{-5}$ micrograms of $I^{125}$ - cDNA complementary to the Legionella Ribosomal RNA.

95 $\mu$l 34.9% ($\frac{W}{V}$) sodium dodecyl sultate in $H_2O$.

2. Incubate the mixture at 72°C and at specified times remove aliquots. Dilute each aliquot and assay for hybridization as described earlier.

This procedure resulted in a rate increase of 150-200 fold over the rate at the reference condition.

EXAMPLE 4

D. Detection of Legionella Bacteria Present in a Liquid Sample.

This example shows that a greatly accelerated hybridization rate occurs at 31.4% ($\frac{W}{V}$) SDS when bacteria are isolated out of a liquid sample and lysed. Accelerated hybridization occurs even in the presence of non-nucleic acid bacterial cell components.

1. (a) Centrifuge a sample known to contain Legionella organisms at 14,000 xg for 10 minutes, and then remove the supernatant.

(b) Resuspend the pellet in a lysing buffer and lyse the bacteria, thus freeing the nucleic acid.

(c) Thoroughly mix:

1 $\mu$l of lysed bacteria solution in 5% sodium dodecyl sulfate, 0.05m Tris buffer pH = 8.2.

1 $\mu$l $H_2O$.

1 $\mu$l 5.0M Sodium Phosphate Buffer (PB)

2 $\mu$l probe solution containing $5 \times 10^{-5}$ micrograms of $I^{125}$ - cDNA complementary to the Legionella Ribosomal RNA.

2. Incubate the mixture at 72°C and at specified times remove aliquots. Dilute each aliquot and assay for hybridization as described earlier.

13

This procedure resulted in a rate increase of 100-200 fold over the rate at the reference condition.

EXAMPLE 5

E. Detection of Legionella Bacteria in a Sputum Sample Known To Contain Such Bacteria.

This example shows that a greatly accelerated hybridization rate occurs at 23.4% ($\frac{W}{V}$) SDS when the bacteria have been isolated out of a clinical sample, lysed and hybridized with no purification of RNA.

1. (a) Solubilize 1 ml sputum by adding 0.1ml of 0.1M DTT and centrifuge for 10' at 14,000 xg to pellet bacteria. Discard the supernatant.

(b) Resuspend pellet in lysing buffer and lyse Legionella bacteria to free nucleic acids.

(c) Thoroughly mix:

30 $\mu$l of lysing solution (containing about 8000 lysed Legionella bacteria) composed of 11% sodium dodecyl sulfate ($\frac{W}{V}$); $3\times10^{-3}$M EDTA; 0.003M Tris unbuffered.

1 $\mu$l 5M Sodium Phosphate Buffer (pH = 6.8) (PB).

2 $\mu$l probe solution containing $10^{-4}$ micrograms of $I^{125}$ - cDNA complementary to the Legionella rRNA.

67 $\mu$l 34.9% ($\frac{W}{V}$) SDS.

2. Incubate the mixture at 72°C and at specified times remove aliquots. Dilute each aliquot and assay for hybridization as described earlier.

This procedure resulted in a rate increase of 100-200 fold over the rate at the reference condition.

EXAMPLE 6

F. Detection of Legionella Bacteria in a Sputum Sample Known to Contain Such Bacteria

This example shows that a greatly accelerated hybridization rate occurs at 23.4% ($\frac{W}{V}$) SDS when the clinical sample is assayed directly and the hybridization is done in the presence of the sputum components.

1. (a) Mix a sputum sample known to contain Legionella bacteria with an equal volume of a lysing agent solution (33% SDS, 0.01m unbuffered Tris, 0.01m EDTA, 0.01m EGTA). Incubate at 72°C 15 minutes.

(b) Mix thoroughly:

30 $\mu$l of solution from 1(a).

1 $\mu$l 5M Sodium Phosphate Buffer (pH = 6.8)(PB).

2 $\mu$l probe solution containing $10^{-4}$ micrograms of of $I^{125}$ - cDNA complementary to the Legionella rRNA.

67 $\mu$l 34.9% ($\frac{W}{V}$) SDS in $H_2O$.

2. Incubate the mixture at 72°C and at specified times remove aliquots. Dilute each aliquot and assay for hybridization as described earlier.

This procedure resulted in a rate increase of 100-200 fold over the rate at the reference condition.

EXAMPLE 7

Method For Using Sodium Tetradecyl Sulfate (STDS) To Incrase Nucleic Acid Hybridization Rate

This example shows a greatly accelerated rate occurs at 24.3% ($\frac{W}{V}$) STDS when purified RNA is used.

A. RNA:DNA Hybridization in STDS.

1. Thoroughly mix:

1 $\mu$l of a solution containing $1.7\times10^{-4}$ micrograms of Legionella rRNA.

7 $\mu$l $H_2O$.

2 $\mu$l probe solution containing $10^{-4}$ micrograms of $I^{125}$ - cDNA complementary to Legionella rRNA.

90 $\mu$l 27% ($\frac{W}{W}$) STDS, 0.03M PB; final pH = 7.

2. Incubate the mixture at 72°C and at specified times remove aliquots. Dilute each aliquot and assay for hybridization as described earlier.

This procedure resulted in a rate increase of at least 500-1000 fold over the rate at the reference condition.

14

EXAMPLE 8

B. RNA:DNA Hybridization in STDS + UREA. Addition of Urea Increases The Extent of Hybridization.

This example shows that a greatly accelerated rate occurs at 24.3% ($\frac{W}{W}$) STDS when Urea is present in the reaction mix.

1. Mix thoroughly:

1 $\mu$l solution containing $1.7 \times 10^{-4}$ micrograms of Legionella rRNA.

2 $\mu$l $H_2O$.

5 $\mu$l 10M Urea.

2 $\mu$l probe solution containing $10^{-4}$ micrograms of $I^{125}$ - cDNA complementary to the Legionella rRNA.

90 $\mu$l 27% ($\frac{W}{W}$) STDS, 0.03M PB: final pH = 7.

2. Incubate the mixture at 72°C and at specified times remove aliquots. Dilute each aliquot and assay for hybridization as described earlier.

This procedure resulted in a rate increase of at least 500-1000 fold over the rate at the reference condition.

In addition a greater extent of hybridization is seen with urea present.

EXAMPLE 9

A. Hybridization Rate Increase Prompted by Sodium Diisobutyl Sulfosuccinate (SDIBSS).

This example shows that a greatly accelerated rate occurs at 41.4% ($\frac{W}{W}$) SDIBSS when phosphate buffer (PB) is used to adjust the SDIBSS pH to about 7.

a. RNA:DNA hybridization

1. Mix well:

1 $\mu$l of a solution containing $1.7 \times 10^{-4}$ micrograms of Legionella rRNA.

2 $\mu$l 5M PB.

2 $\mu$l $I^{125}$ - cDNA complementary Legionella rRNA

4 $\mu$l $H_2O$.

92 $\mu$l 45% ($\frac{W}{W}$) SDIBSS.

2. Incubate at 72°C and at specified times remove aliquots. Dilute each aliquot and assay for hybridization as described earlier.

This procedure resulted in a rate increase of 100-200 fold over the rate at the reference condition.

EXAMPLE 10

B. RNA:DNA Hybridization in SDIBSS

This example shows that a greatly accelerated rate occurs at 33.8% ($\frac{W}{W}$)SDIBSS when phosphate buffer is used to adjust the SDIBSS pH to about 7.

1. Mix well:

1 $\mu$l solution containing $1.7 \times 10^{-4}$ micrograms of Legionella rRNA.

2 $\mu$l 5M PB.

2 $\mu$l $I^{125}$ - cDNA complementary to Legionella rRNA.

20 $\mu$l $H_2O$

75 $\mu$l SDIBSS 45% ($\frac{W}{W}$).

2. Incubate at 72°C and at specified times remove aliquots. Dilute each aliquot and assay it for hybridization as described earlier.

This procedure results in a hybridization rate increase of 100-200 fold over the reference condition rate.

EXAMPLE 11

C. RNA:DNA Hybridization in SDIBSS

This example shows that a greatly accelerated rate occurs at 38.2% ($\frac{W}{W}$)SDIBSS when phosphate buffer is used to adjust the SDIBSS pH to about 7.

15

1. Mix well:

   1 μl of solution containing 1.7x10$^{-4}$ micrograms <u>Legionella</u> rRNA.

   2 μl 5M PB

   2 μl I$^{125}$ - cDNA complementary to <u>Legionella</u> rRNA.

   10 μl H$_2$O.

   85 μl 45% ($\frac{w}{w}$) SDIBSS.

2. Incubate at 72°C and at specified times remove aliquots. Dilute each aliquot and assay it for hybridization as described earlier.

This procedure results in a hybridization rate increase of 100-200 fold over the reference condition rate.

EXAMPLE 12

D. RNA:DNA Hybridization in SDIBSS.

A greatly accelerated rate occurs when unbuffered Tris is used to adjust the SDIBSS pH to about 9.

1. Mix well:

   1 μl of solution containing 1.7x10$^{-4}$ micrograms of <u>Legionella</u> rRNA.

   7 μl 1M Tris, unbuffered.

   2 μl I$^{125}$ - cDNA complementary to <u>Legionella</u> rRNA.

   90 μl 45% ($\frac{w}{w}$) SDIBSS.

2. Incubate the mixture at 72°C and at specified times remove aliquots. Dilute each aliquot and assay it for hybridization as described earlier.

This procedure resulted in a rate increase of 100-200 fold over the rate at the reference condition.

EXAMPLE 13

E. RNA:DNA Hybridization In A Mixture of Sodium Dodecyl Sulfate (SDS) and SDIBSS

A greatly accelerated rate occurs in a mixture of SDS and SDIBSS.

1. Mix well:

   1 μl of solution containing 1.7x10$^{-4}$ micrograms <u>Legionella</u> rRNA.

   7 μl 1M Tris unbuffered.

   2 μl I$^{125}$ - cDNA complementary to <u>Legionella</u> rRNA.

   45 μl 34.9% ($\frac{w}{v}$) SDS.

   45 μl 45% ($\frac{w}{v}$) SDIBSS.

2. Incubate the mixture at 72°C and at specified times remove aliquots. Dilute each aliquot and assay it for hybridization as described earlier.

This procedure resulted in a rate increase of 100-200 fold over the rate at the reference condition.

EXAMPLE 14

F. RNA:DNA Hybridization In SDIBSS In The Presence of Heterologous RNA

A greatly accelerated rate occurs in the presence of a high concentration (56 micrograms/ml) of heterologous RNA.

1. Mix well:

   1 μl of solution containing 1.7x10$^{-4}$ micrograms <u>Legionella</u> rRNA.

   3 μl of solution containing 5.6 micrograms of heterologous bacterial RNA.

   4 μl 1M Tris, unbuffered.

   2 μl I$^{125}$ - cDNA complementary to <u>Legionella</u> rRNA.

   90 μl 45% ($\frac{w}{w}$) SDIBSS.

2. Incubate the mixture at 72°C and at specified times remove aliquots. Dilute each aliquot and assay it for hybridization as described earlier.

This procedure resulted in a rate increase of at least 100-200 fold over the rate at the reference condition.

EXAMPLE 15

16

EP 0 229 442 B1

G. Detection of Legionella Bacteria In Sputum By RNA:DNA Hybridization In SDIBSS.

A greatly accelerated rate occurs when bacteria are isolated from sputum, lysed and hybridized in SDIBSS. Hybridization occurred in SDIBSS even in the presence of non-nucleic acid cellular components.

1. (a) Solubilize 1 ml of sputum by adding 0.1ml 0.25, DTT and centrifuge the mixture at 14,000 x g for 10' to pellet bacteria. Discard supernatant.

(b) Resuspend pellet in one third of its volume of lysing buffer (33% SDS, 0.01M EDTA, 0.01M Tris, unbuffered) and incubate at 72°C for 15' to lyse bacteria.

(c) Mix well:

2 $\mu$l 5M PB.

4 $\mu$l I$^{125}$ - cDNA complementary to Legionella rRNA.

30 vi 45% ($\frac{W}{W}$) DBISS.

(d) Add mixture (c) to mixture (b) and thoroughly mix.

2. Incubate the mixture at 72°C and at specified times remove aliquots. Dilute each aliquot and assay it for hybridization as described earlier.

This procedure resulted in a rate increase of 100-200 fold over the rate of reference condition.

EXAMPLE 16

H. Detection of Legionella Bacteria In A Liquid Sample By RNA:DNA Hybridization In SDIBSS.

A greatly accelerated rate occurs when the bacteria are isolated from a non-clinical sample and hybridized in SDIBSS. A separate lysing incubation step was not performed.

1. (a) Prefilter the liquid sample known to contain Legionella to remove large particles.

(b) Mix well:

30 $\mu$l solution of solution containing Legionella bacteria.

15 $\mu$l 33% SDS, 0.01M Tris, unbuffered, 0.01M EDTA.

1.8 $\mu$l 5M PB.

5 $\mu$l I$^{125}$ - cDNA complementary to Legionella rRNA.

190 $\mu$l 45% ($\frac{W}{W}$) SDIBSS.

2. Incubate at 72°C and at specified times remove aliquots. Dilute each aliquot and assay it for hybridization as described earlier.

This procedure results in a hybridization rate increase of 100-200 fold over the reference condition rate.

EXAMPLE 17

I. RNA:DNA Hybridization In The Presence of SDIBSS and Urea.

A greatly accelerated rate occurs in a mixture of SDIBSS and Urea.

1. Mix well:

1 $\mu$l of solution containing 1.7x10$^{-4}$ micrograms of Legionella rRNA.

2 $\mu$l H$_2$O.

5 $\mu$l 10M Urea.

90 $\mu$l SDIBSS 45% ($\frac{W}{W}$).

2. Incubate at 72°C and at specified times remove aliquots. Dilute each aliquot and assay it for hybridization as described earlier.

This procedure results in a hybridization rate increase of at least 200 fold over the reference condition rate.

EXAMPLE 18

J. RNA:DNA Hybridization In SDIBSS and Urea

A greatly accelerated rate occurs in a mixture of SDIBSS and Urea.

1. Mix well:

2 $\mu$l solution containing 3.4x10$^{-4}$ micrograms of Legionella rRNA.

1 $\mu$l 5M PB.

2 $\mu$l I$^{125}$ - cDNA complementary to Legionella rRNA.

17

25 $\mu$l 27% ( $\frac{W}{W}$ ) SDIBSS, 8M Urea.

70 $\mu$l 45% ( $\frac{W}{W}$ ) SDIBSS.

2. Incubate at 72°C and at specified times remove aliquots. Dilute each aliquot and assay it for hybridization as described earlier.

This procedure results in a hybridization rate increase of 100-200 fold over the reference condition rate.

EXAMPLE 19

K. RNA:DNA Hybridization In The Presence Of SDIBSS and NaSCN

A greatly accelerated rate occurs in a mixture of SDIBSS and sodium thiocyanate.

1. Mix well:

2 $\mu$l solution containing 3.4x10$^{-4}$ micrograms of Legionella rRNA.

1 $\mu$l 5M PB.

2 $\mu$l I$^{125}$ - cDNA complementary to Legionella rRNA.

5 $\mu$l 10.5M NaSCN.

90 $\mu$l 45% ( $\frac{W}{W}$ ) SDIBSS.

2. Incubate at 72°C and at specified times remove aliquots. Dilute each aliquot and assay it for hybridization as described earlier.

This procedure results in a hybridization rate increase of 100-200 fold over the reference condition rate.

EXAMPLE 20

L. RNA:DNA Hybridization In Mixtures Of SDIBSS, Urea and NaSCN.

A greatly accelerated rate occurs in the presence of SDIBSS, sodium thiocyonate and Urea.

1. Mix well:

2 $\mu$l of solution containing 3.4x10$^{-4}$ micrograms of Legionella rRNA.

1 $\mu$l 5M PB.

2 $\mu$l I$^{125}$ - cDNA complementary to Legionella rRNA.

5 $\mu$l 4M Urea, 4M NaSCN , 0.05 M DTT, 0.03M Hcl.

90 $\mu$l 45% ( $\frac{W}{W}$ ) SDIBSS.

2. Incubate at 72°C and at specified times remove aliquots. Dilute aliquot and assay it for hybridization as described earlier.

This procedure results in a hybridization rate increase of at least 100-200 fold over the reference condition rate.

The following examples concern the rapid and sensitive detection of three medically important bacteria, each of which causes disease in humans.

A. Bacteria in the Mycobacteria group cause a variety of human disease. Prominent among them are tuberculosis and leprosy. Current diagnostic methods use the culture approach for the detection and identification of these bacteria. A growth step is necessary to amplify the bacteria so they can be detected and differential growth methods are used to identify these bacteria. This method of diagnosis is labor intensive and very slow. As it is important to know as soon as possible if the patient is infected with Mycobacteria in order to start the proper anti-microbial therapy, this method of diagnosis is labor intensive and very slow.

With current methods it takes 1-8 weeks to obtain a definitive diagnosis for Mycobacteria. The assay described in Example 25 takes about 2-3 hours to perform and does not require a growth step. Thus the method of the invention allows the design of a test for Mycobacteria which is over 100 times faster than the current methods, does not require a growth step, is much less labor intensive and is less expensive. In addition, the rapidity of the test will make rapid treatment of the disease possible with incalculable benefits to the patients.

B. The bacteria Mycoplasma pneumoniae also causes disease in humans. Culture methods are currently used for diagnosis and a definitive diagnosis generally takes from 1-2 weeks.

The assay described in Example 23 takes less than 2 hours to perform and does not require a growth step. Thus the method of the invention allows the design of a test for Mycoplasma pneumoniae which is about 100 times faster than the current methods and has other advantages and benefits similar to those described in A above.

C. Legionella bacteria also cause human disease. Current recommended diagnostic procedures involve

EP 0 229 442 B1

culture methods. Such methods generally yield a definitive answer in about 3 days but can take a week or longer.

The assay described in Example 25 takes less than 2 hours to perform, does not require a growth step, is about 20 times faster than current methods and has other advantages and benefits similar to those described in A above.

EXAMPLE 21

M. Detection Of Legionella Bacteria In Sputum Sample By RNA:DNA Hybridization In SDIBSS, Urea and SDS.

A greatly accelerated rate occurs when sputum is assayed directed in a mixture of SDIBSS, SDS and Urea.

1. (a) Mix well:

3 $\mu$l sputum.

6 $\mu$l 17% SDS, 0.01M Tris (unbuffered), 0.01M EDTA. Incubate at 72°C for 15'.

(b) Mix well:

3 $\mu$l 1(a) solution.

2 $\mu$l $H_2O$.

3 $\mu$l 10M Urea.

2 $\mu$l $I^{125}$ - cDNA complementary to Legionella rRNA.

90 $\mu$l 45% ($\frac{W}{W}$) SDIBSS.

2. Incubate at 72°C and at specified times remove aliquots. Dilute each aliquot and assay it for hybridization as described earlier.

This procedure results in a hybridization rate increase of 100-200 fold over the reference condition rate.

EXAMPLE 22

N. Detection of Legionella Bacteria In Sputum Sample By RNA:DNA Hybridization In SDIBSS, Urea, SDS Mixes.

An example of the rapid detection of Legionella bacteria in a sputum sample by using a mixture of SDIBSS, SDS, and Urea to greatly accelerate hybridization rate.

1. (a) Mix well.

20 $\mu$l of sputum.

80 $\mu$l 5M Urea, 4M NaSCN, 0.05M Tris (unbuffered) and centrifuge for 10 minutes at 14,000 x g. Discard supernate.

(b) Add 25 $\mu$l 11% SDS, 3.3M Urea, 0.007M EDTA, 0.05M Tris (unbuffered) to pellet and resuspend. Incubate at 72°C for 15 minutes.

(c) Mix well:

5 $\mu$l solution for 1(b).

2 $\mu$l $I^{125}$ - cDNA complementary to Legionella rRNA.

3 $\mu$l 10M Urea

90 $\mu$l 45% ($\frac{W}{W}$) SDIBSS.

2. Incubate the mixture at 72°C for one hour and assay for hybridization as described.

This procedure resulted in a rate increase of 100-200 over the rate at the reference condition.

EXAMPLE 23

O. Detection of Mycoplasma Pneumonia Bacteria In A Throat Swab Sample By RNA:DNA Hybridization In SDIBSS.

An example of the use of the SDIBSS system for rapid detection of Mycoplasma pneumoniae in a clinical sample.

1. (a) Resuspend throat swab material in a suitable solution which is compatible with the bacteria of interest.

(b) Centrifuge the solution at 13,000 x g for 10 minutes. Discard supernatant.

(c) Resuspend the pellet in 300 $\mu$l of 45% ($\frac{W}{W}$) SDIBSS containing 3% SDS, 0.03M PB, (PH = 6.8),

19

$10^{-3}$M EDTA, $10^{-3}$M EDTA, $10^{-3}$M EGTA and $I^{125}$ - cDNA complementary to M. pneumonia rRNA.

2. Incubate the mixture at 72°C for one hour and assay for hybridization as described.

This procedure resulted in a rate increase of 100-200 fold over the rate at the reference condition.

EXAMPLE 24

A. Detection Of The Presence Of Mycoplasma In A Tissue Culture By Means Of RNA:DNA Hybridization In SDIBSS.

An example of the use of the Sodium Phosphate system for the rapid detection of Mycoplasma infection of tissue culture is disclosed.

1. (a) Centrifuge 50 $\mu$l of tissue culture media from a baby hamster kidney cell culture for 5 minutes at 12,000 x g. Discard the supernatant.

(b) Resuspend the pellet in 100 $\mu$l of 0.15M NaCl.

(c) Mix well:

45 $\mu$l solution (b).

5 $\mu$l 5% Sarkosyl, $10^{-2}$M EDTA, $10^{-2}$M EGTA, 0.96M PB, 0.01 micrograms/ml of 3H-cDNA complementary to Mycoplasma hominis rRNA.

65 $\mu$l 4.8M PB.

and sufficient SDIBSS to greatly accelerate the rate of hybridization.

2. Incubate at 72°C for one hour and assay for hybridization as described.

This procedure results in a hybridization rate increase of 100-200 fold over the reference condition rate.

EXAMPLE 25

B. Detection Of Mycobacteria In A Sputum Sample By RNA:DNA Hybridization In SDIBSS.

This is an example of the use of the SDIBSS system for the rapid detection of Mycobacteria in a clinical sample.

1. (a) Liquefy sputum and centrifuge solution to pellet Mycobacteria discard supernatant.

(b) Suspend pellet in 40 $\mu$l of 3.3% SDS and add 50 $\mu$l of glass beads (0.2-0.3mm)(Dyno-Mill brand), sonicate 10 minutes in a mettler M4 ultrasonic cleaner.

(c) Mix well:

Solution from b).

100 $\mu$l 45% ($\frac{W}{W}$) SDIBSS.

176 $\mu$l 43.5% ($\frac{W}{W}$) SDIBSS, 0.07M PB, $I^{125}$-cDNA ($10^{-2}$ micrograms/ml) complementary to Mycobacteria rRNA.

2. Incubate the mixture at 72°C for one hour and assay for hybridization as described.

This procedure resulted in a rate increase of 100-200 fold over the rate at the reference condition.

EXAMPLE 26

Detection Of Legionella Bacteria In A Liquid Sample By RNA:DNA Hybridization In SDIBSS and Amphyl (Sold By National Laboratories). Active Ingredients In Amphyl, 10.5% 0-Phenylphenol, 5% 0-Benzo-P-Chlorophenol, 84.5% Inert Ingredients .

A greatly accelerated rate occurs in a mixture of Sodium Phosphate and Amphyl, a bactericidal agent.

1. Mix well:

2 $\mu$l solution containing $1.2 \times 10^4$ intact Legionella bacteria.

12 $\mu$l solution containing 4% Amphyl and radioactive cDNA complementary to Legionella rRNA.

18 $\mu$l 4.8M PB

and sufficient SDIBSS to greatly accelerate the rate of hybridization.

2. Incubate at 72°C and at specified times remove aliquots. Dilute each aliquot and assay it for hybridization as described earlier.

This example demonstrates that the rRNA of Legionella bacteria is made available for hybridization by adding Amphyl to the reaction mixture. No pre-cracking of the bacteria is necessary.

**Claims**

20

1. A method for the formation of double-stranded nucleic acid molecules from separate single-stranded nucleic acid molecules wherein the rate of reaction is greatly increased over the standard reference condition reaction rate, used for detecting the presence or amount of bacteria in a sample suspected of containing bacteria, said method comprising:

preparing an aqueous reaction solution containing a quantity of a test sample suspected of containing bacteria, said test sample having been treated with a quantity of denaturing agent sufficient to disassociate the double-stranded nucleic acid molecules present in said bacteria into single-stranded nucleic acid molecules, a quantity of a second single-stranded nucleic acid molecule complementary to a segment of the base sequence of the nucleic acid molecules of the organism to be detected, and a known concentration of at least one nucleic acid precipitating agent detergent, said known concentration being sufficient to accelerate the rate of reaction to 100 or more times the rate of the standard reference condition reaction;

incubating said aqueous reaction solution at a temperature at which reassociation can occur; and

assaying said incubated aqueous reaction solution for the presence or amount of double-stranded nucleic acid molecules of the bacteria to be detected.

2. A method according to claim 1 in which the detergent comprises one or more of sodium dodecyl sulfate (SDS), sodium diisobutyl sulfosuccinate (SDIBSS) or sodium tetradecylsulfate (STDS).

3. A method of claim 1 or 2 wherein said reaction solution contains a denaturing agent in a concentration which does not significantly inhibit said accelerated rate of reassociation.

4. A method of claims 1, 2 or 3 wherein said reaction solution contains urea, NaSCN, guanidine HCl.

5. A method of any one of the preceding claims wherein said reaction solution contains less than about 2M urea, or less than about 2M NaSCN.

6. A method of any one of the preceding claims wherein said bacteria are a member of the genus Legionella.

7. A method of any of claims 1 to 5 wherein said bacteria are a member of the group Mycobacterium.

8. A method of any one of claims 1 to 5 where said bacteria is Mycoplasma pneumoniae.

9. A method of any one of the preceding claims where said sample is a clinical sample.

10. A method of claim 9 wherein said clinical sample includes sputum or a throat swab.

11. A method of any one of the preceding claims wherein said reaction solution includes a bactericidal agent.

12. A method for assaying for the presence or amount of Legionella bacteria in a clinical sample suspected of containing Legionella bacteria, said method comprising:

preparing an aqueous reaction solution containing a quantity of said test sample having been optionally treated to denature its double stranded nucleic acid molecules to single-stranded form,

cDNA complementary to a segment of Legionella rRNA,

SDIBSS in sufficient concentration to accelerate the rate of hybridization to 100 or more times over the rate (if any) of standard reference condition reaction, and

a bactericidal agent;

incubating said aqueous reaction solution at about 72°C for a time sufficient for said cDNA to substantially hybridize to complementary bacterial nucleic acid present in the clinical sample; and

assaying said aqueous reaction solution for the presence or amount of hybridization.

13. A method for assaying for the presence or amount of Mycoplasma pneumoniae in a clinical sample suspected of containing said M. pneumoniae, said method comprising:

preparing an aqueous reaction solution containing

a quantity of said test sample having been optionally treated to denature its double stranded

21

nucleic acid molecules to single stranded form,

cDNA complementary to a segment of Mycoplasma pneumoniae rRNA,

SDIBSS in sufficient concentration to accelerate the rate of hybridization to 100 or more times over the rate (if any) of standard reference condition reaction;

incubating said aqueous reaction solution at about 72ºC for a time sufficient for said cDNA to substantially hybridize to complementary bacterial nucleic acid present in the clinical sample; and,

assaying said aqueous reaction solution for the presence or amount of hybridization.

**Patentansprüche**

1. Verfahren zur Bildung doppelsträngiger Nucleinsäuremoleküle aus separaten einzelsträngigen Nucleinsäuremolekülen, bei dem die Reaktionsgeschwindigkeit gegenüber der Reaktionsgeschwindigkeit unter Standardbedingungen beträchtlich erhöht ist, das zum Nachweis der Gegenwart oder der Menge von Bakterien in einer Probe, die im Verdacht steht, Bakterien zu enthalten, verwendet wird, umfassend:

das Herstellen einer wäßrigen Reaktionslösung, die eine Menge einer Testprobe enthält, die im Verdacht steht, Bakterien zu enthalten, wobei die Testprobe mit einer Menge von denaturierendem Agens behandelt worden ist, die ausreicht, um die doppelsträngigen Nucleinsäuremoleküle, die in den Bakterien vorhanden sind, in einzelsträngige Nucleinsäuremoleküle zu dissoziieren, einer Menge eines zweiten einzelsträngigen Nucleinsäuremoleküls, das zu einem Segment der Basensequenz der Nucleinsäuremoleküle des nachzuweisenden Organismus komplementär ist, und einer bekannten Konzentration von mindestens einem Nucleinsäure ausfällenden Detergens, wobei die bekannte Konzentration ausreicht, um die Reaktionsgeschwindigkeit auf das 100- oder mehrfache der Geschwindigkeit der Reaktion unter Standardbedingungen zu beschleunigen;

das Inkubieren der wäßrigen Reaktionslösung bei einer Temperatur, bei der Reassoziation stattfinden kann; und

das Testen der inkubierten wäßrigen Reaktionslösung auf die Gegenwart oder die Menge doppelsträngiger Nucleinsäuremoleküle der nachzuweisenden Bakterien.

2. Verfahren nach Anspruch 1, bei dem das Detergens Natriumdodecylsulfat (SDS), Natrium-diisobutyl-sulfosuccinat (SDIBSS) und/oder Natrium-tetradecylsulfat (STDS) umfaßt.

3. Verfahren nach Anspruch 1 oder 2, bei dem die Reaktionslösung ein denaturierendes Agens in einer Konzentration enthält, die die Beschleunigungsrate der Reassoziation nicht signifikant inhibiert.

4. Verfahren nach Ansprüchen 1, 2 oder 3, bei dem die Reaktionslösung Harnstoff, NaSCN, Guanidin HCl enthält.

5. Verfahren nach einem der vorhergehenden Ansprüche, worin die Reaktionslösung weniger als etwa 2 M Harnstoff oder weniger als etwa 2 M NaSCN enthält.

6. Verfahren nach einem der vorhergehenden Ansprüche, worin die Bakterien ein Mitglied der Gattung Legionella sind.

7. Verfahren nach einem der Ansprüche 1-5, worin die Bakterien ein Mitglied der Gruppe Mycobakterium sind.

8. Verfahren nach einem der Ansprüche 1-5, worin die Bakterien Mycoplasma pneumoniae sind.

9. Verfahren nach einem der vorhergehenden Ansprüche, worin die Probe eine klinische Probe ist.

10. Verfahren nach Anspruch 9, worin die klinische Probe Sputum oder einen Rachenabstrich enthält.

11. Verfahren nach einem der vorhergehenden Ansprüche, worin die Reaktionslösung ein bakterizides Agens enthält.

**12.** Verfahren zum Testen der Gegenwart oder der Menge von Legionella-Bakterien in einer klinischen Probe, die im Verdacht steht, Legionella-Bakterien zu enthalten, umfassend:

das Herstellen einer wäßrigen Reaktionslosung, die eine Menge der Testprobe enthält, die ggfs. behandelt wurde, um ihre doppelsträngigen Nucleinsäuremoleküle zur Einzelstrangform zu denaturieren,

cDNA, die komplementär zu einem Segment von Legionella-rRNA ist,

SDIBSS in ausreichender Konzentration, um die Hybridisierungsgeschwindigkeit auf das 100- oder mehrfache der Geschwindigkeit (falls vorhanden) der Reaktion unter Standardbedingungen zu beschleunigen, und

ein bakterizides Agens;

Inkubieren der wäßrigen Reaktionslösung bei etwa 72°C für eine Zeit, die für die cDNA ausreichend ist, um im wesentlichen mit in der klinischen Probe vorhandener komplementärer Bakteriennucleinsäure zu hybridisieren; und

Testen der wäßrigen Reaktionslösung auf das Vorliegen von oder die Menge an Hybridisierung.

**13.** Verfahren zum Testen der Gegenwart oder der Menge von Mycoplasma pneumoniae in einer klinischen Probe, die im Verdacht steht, M. pneumoniae zu enthalten, umfassend:

das Herstellen einer wäßrigen Reaktionslösung, die eine Quantität der Testprobe enthält, die ggfs. behandelt wurde, um ihre doppelsträngigen Nucleinsäuremoleküle in die Einzelstrangform zu denaturieren,

cDNA, die komplementär zu einem Segment von Mycoplasma pneumoniae-rRNA ist;

SDIBSS in ausreichender Konzentration, um die Hybridisierungsgeschwindigkeit auf das 100- oder mehrfache der Geschwindigkeit (falls vorhanden) der Reaktion bei Standardbedingungen zu beschleunigen;

Inkubieren der wäßrigen Reaktionslosung bei etwa 72°C für eine Zeit, die für die cDNA ausreichend ist, um im wesentlichen mit in der klinischen Probe vorhandener komplementärer Bakteriennucleinsäure zu hybridisieren; und

Testen der wäßrigen Reaktionslösung auf die Gegenwart von oder die Menge der Hybridisierung.

**Revendications**

**1.** Procédé de formation de molécules d'acide nucléique à deux brins à partir de molécules d'acide nucléique mono brin séparées, dans lequel la vitesse de réaction est fortement accrue par rapport à la vitesse de réaction dans les conditions de référence standard, utilisée pour détecter la présence ou la quantité de bactéries dans un échantillon dont on soupçonne qu'il contient des bactéries, ledit procédé comprenant :

La préparation d'une solution réactionnelle aqueuse contenant une certaine quantité d'un échantillon à étudier dont on soupçonne qu'il contient des bactéries, ledit échantillon à étudier ayant été traité avec une quantité d'agent dénaturant suffisante pour désassocier les molécules d'acide nucléique à deux brins présentes dans lesdites bactéries en molécules d'acide nucléique mono brin, une certaine quantité d'une seconde molécule d'acide nucléique mono brin complémentaire d'un segment de la séquence de base des molécules d'acide nucléique de l'organisme à détecter, et au moins un détergent agent précipitant d'acide nucléique à une concentration connue, ladite concentration connue étant suffisante pour accélérer la vitesse de réaction jusqu'à une vitesse de réaction égale ou supérieure à 100 fois celle dans les conditions de référence standard ;

l'incubation de ladite solution réactionnelle aqueuse à une température à laquelle une réassociation peut se produire ; et

EP 0 229 442 B1

le dosage de ladite solution réactionnelle aqueuse incubée pour déterminer la présence ou la quantité de molécules d'acide nucléique à deux brins des bactéries à détecter.

2. Procédé selon la revendication 1 dans lequel le détergent comprend un ou plusieurs des corps suivants : docécyl-sulfate de sodium (SDS), diisobutyl-sulfo-succinate de sodium (SDIBSS) ou tétradécylsulfate de sodium (STDS).

3. Procédé de la revendication 1 ou 2 dans lequel ladite solution réactionnelle contient un agent dénaturant à une concentration qui n'inhibe pas sensiblement ladite vitesse accélérée de réassociation.

4. Procédé des revendications 1, 2 ou 3 dans lequel ladite solution réactionnelle contient de l'urée, NaSCN, du chlorhydrate de guanidine.

5. Procédé de l'une quelconque des revendications précédentes dans lequel ladite solution réactionnelle contient de l'urée dans une quantité inférieure à environ 2 M, ou NaSCN dans une quantité inférieure à environ 2 M.

6. Procédé de l'une quelconque des revendications précédentes dans lequel lesdites bactéries appartiennent au genre Legionella.

7. Procédé de l'une quelconque des revendications 1 à 5 dans lequel lesdites bactéries appartiennent au groupe Mycobacterium.

8. Procédé de l'une quelconque des revendications 1 à 5 dans lequel ladite bactérie est Mycoplasma pneumoniae.

9. Procédé de l'une quelconque des revendications précédentes dans lequel ledit échantillon est un échantillon clinique.

10. Procédé de la revendication 9 dans lequel ledit échantillon clinique inclut un crachat ou un prélèvement fait dans la gorge avec un tampon d'ouate.

11. Procédé de l'une quelconque des revendications précédentes dans lequel ladite solution réactionnelle inclut un agent bactéricide.

12. Procédé de dosage pour détecter la présence ou la quantité de bactéries Legionella dans un échantillon clinique dont on soupçonne qu'il contient des bactéries Legionella, ledit procédé comprenant :
la préparation d'une solution réactionnelle aqueuse contenant une certaine quantité dudit échantillon à étudier éventuellement traité pour dénaturer ses molécules d'acide nucléique à deux brins en forme de mono brin,
l'ADNc complémentaire d'un segment d'ARNr de Legionella,
du SDIBSS en concentration suffisante pour accélérer la vitesse d'hybridation jusqu'à une vitesse de réaction égale ou supérieure à 100 fois celle dans les conditions de référence standard (s'il y en a une), et
un agent bactéricide ;
l'incubation de ladite solution réactionnelle aqueuse à environ 72° C pendant une durée suffisante pour que ledit ADNc s'hybride sensiblement à l'acide nucléique bactérien complémentaire présent dans l'échantillon clinique ; et
le dosage de ladite solution réactionnelie aqueuse pour détecter la présence ou la quantité d'hybridation.

13. Procédé de dosage pour détecter la présence ou la quantité de Mycoplasma pneumoniae dans un échantillon clinique dont on soupçonne qu'il contient ladite M. pneumoniae, ledit procédé comprenant :
la préparation d'une solution réactionnelle aqueuse contenant une certaine quantité dudit échantillon à étudier préalablement éventuellement traitée pour dénaturer ses molécules d'acide nucléique à deux brins en forme de mono brin,
l'ADNc complémentaire d'un segment d'ARNr de Mycoplasma pneumoniae,

24

Mycoplasma pneumoniae,

du SDIBSS à une concentration suffisante pour accélérer la vitesse d'hybridation jusqu'à une vitesse de réaction égale ou supérieure à 100 fois celle dans les conditions de référence standard (s'il y en a une) ;

l'incubation de ladite solution réactionnelle aqueuse à environ 72° C pendant une durée suffisante pour que ledit ADNc s'hybride sensiblement à l'acide nucléique bactérien complémentaire présent dans l'échantillon clinique ; et,

le dosage de ladite solution réactionnelle aqueuse pour détecter la présence ou la quantité d'hybridation.